# EUROPEAN PATENT SPECIFICATION

(11) **EP 2 582 592 B1**
(45) Date of publication and mention of the grant of the patent: **27.01.2016**
(21) Application number: 10747500.6
(22) Date of filing: 28.06.2010
(51) Int. Cl.: B65D 33/02, A61F 15/00, B65D 75/58, B65D 75/08, B65B 25/14, B65D 33/08

(54) **PACKAGING BAG OF FILM MATERIAL FOR PACKAGING PRODUCTS REMOVABLE THROUGH AN OPENING COVERED BY A FLAP INTEGRATED TO THE PACKAGING BAG, AND RELATIVE MANUFACTURING METHOD**
FOLIENVERPACKUNGSBEUTEL MIT EINER IM BEUTEL INTEGRIERTEN LASCHE, DIE EINE ÖFFNUNG ZUR ENTNAHME VON PRODUKTEN DECKT, UND VERFAHREN DAZU
SACHET D'EMBALLAGE EN FILM POUR EMBALLER DES PRODUITS CAPABLES À ÊTRE RETIRÉS À TRAVERS UNE OUVERTURE COUVERTE PAR UN RABAT INTEGRÉ DANS L'EMBALLAGE, ET PROCÉDÉ POUR LA MANUFACTURE DU SACHET

(30) Priority: 16.06.2010 IT MI20101088
(43) Date of publication of application: 24.04.2013
(73) Proprietor: Joeplast S.p.A., 92025 Castel Termini (AG) (IT)
(72) Inventor: MESSINA, Sergio, 92025 Catel Termini (AG) (IT)
(74) Representative: Guella, Paolo
(86) International application number: PCT/IT2010/000285
(87) International publication number: WO 2011/158265

(56) References cited:
- WO-A1-93/16929
- WO-A1-2006/070771
- US-A- 4 966 286
- US-A1- 2008 152 264

## Description

### Field of application of the invention

The present invention concerns the field of packaging, in the present case a packaging bag of film material for packaging products removable through an opening covered by a flap integrated to the packaging bag, and relative manufacturing method.

### Review of the known art

The technical field referred to above includes many forms of packaging a wide range of products, such as:
- toilet paper rolls, kitchen paper rolls and similar kinds in the so-called tissue field;
- children's nappies, table napkins, and others in the field of health and hygiene;
- cutlery, glasses, plates, etc. in the field of disposable products;
- sandwiches, brioches, biscuits, cakes etc:, in the alimentary field;
- loose vegetables of many kinds, in the field of natural products;
- vacuum-packed products.

The term "packaging" usually means both the container itself and the product packed in it. The term packaging, in its wider sense, includes the science, art and technology of enclosing and protecting products, namely the action and the result of packaging a product. According to a narrower interpretation, this term refers to the only product used for containing and protecting certain types of goods.

Containers made of flexible and easily folded material such as plastic film, paper or vegetable fibres, generally appear, prior to filling, in a flattened shape as that generally assumed by: envelopes, bags, sachets, etc., while, when filled, the shape they acquire depends to a great extent on the type of product put into them.

In packing of hygiene products, the packaging at present on the market consists, for example, of a rectangular sheet of plastic film folded on itself to form two matching parts joined on two parallel sides, to leave the side opposite to the fold open for subsequent filling. One of the two matching parts is longer than the other, the extra length used to form a flap containing two holes for fork-lifting. On the side opposite the fork-lifting flap there is usually a handle, eventually strengthened according to the use made of the bag when complete and therefore to its weight when full. Additionally, on the walls of an ordinary plastic film bag there are one or more holes of about one centimetre, cut without removing any material, of varying shapes such as circular, star or cross-shaped, that serve to allow air to escape during the filling stage. Further, in proximity to the open side and to the two parallel joined sides, two pre-cuts are made of about one centimetre per side, known as anti-shock cuts, their purpose being to absorb the shock inflicted by electro-mechanical systems used for opening the bag and then filling it. There is also a tear-off line on the bag that serves to open it without damage and to permit removal of the content.

Document US-A-2008 152264 which is regarded as the closest prior art discloses a flexible packaging bag with the features in the preamble of claim 1.

With reference to **Figures 1** and **2**, a first example is given by international patent application WO 2008/104444 A1 which, after amendment, claims a packaging 1 for hygiene products 2, said packaging 1 comprising, in order to define a volume accommodating the hygiene products 2:
- at least two main walls 3 which are situated substantially opposite to one another, in which, on one upper side 4 of the packaging 1 opposite a lower side 5, a superimposed part 6 is created by joining or gluing together the two main walls 3;
- a handle 7 provided in the superimposed part 6;
- a tear tab 8 including a section of each of the two main walls 3 and a section of one side wall 9, said tear tab 8 exposing a removal opening (not seen in the figure) for removing hygiene products 2, wherein the tear tab 8 has a grip section 10 for gripping said tear tab 8,
characterized in that the tear tab 8 is formed by two lines of weakness 11, one of which extending in one main wall 3 and the other extending in the other main wall 3, wherein the two lines of weakness 11 meet in the region of the superimposed part 6 of the main walls 3, wherein the lines of weakness 11 extend in the main walls 3 in an angular or curved, in particular arc-shaped manner, wherein the tear tab 8 is attached to the packaging 1 at least in the region of the side wall 9, wherein the grip section 10 comprises at least one opening 12 through which the fingers can be placed, and wherein said packaging 1 accommodates at least two uncompressed hygiene products 2 wound onto rolls, in particular toilet paper rolls, kitchen paper rolls, or household paper rolls. The tear tab 8 comprises an adhesive strip 13 for re-closing the opening.

It has been said that the packaging 1 is substantially made of a film material, preferably transparent, and is joined or glued together at least in the region of the superimposed part 6 and on the lower side 5, so that the film material surrounds the hygiene products packed into the volume defined by the packaging. When, after being pulled, the tear tab 8 remaining attached to the side wall only 9 of the bag, it would tend to sink down along the walls not held together by the adhesive strip 13. For this reason a re-closing system such as this is somewhat inadequate for resisting pressure from the compressed rolls inside the bag, especially if the direction of compression intercepts the tear tab 8 and if the rolls along that direction are of a smaller size than the opening made by pulling the tear tab, in which case they would tend to cause the packaging to tear prematurely. The packaging is also specifically made for containing hygiene products so shaped and so piled that they will possibly form a parallelepiped, and possibly have rounded edges; this would limit the intrinsic capacity of the in-film packaging to adapt to a different shape of packaged product, while respecting the limits imposed by the weldings along the upper and lower sides of the package. For example, packaging made of film material as above, whether or not there are prepared tear-off lines, could perfectly well be used for packaging vegetable products of different shapes, such as: peas or beans (unshelled or shelled), walnuts, bananas, etc., in which case it is unlikely that the package could assume a parallelepiped form, but in a case of maximum tightening it would be more or less cylindrical, at least in its central part. At any rate the form would be such that it would be impossible to distinguish two main walls one opposite the other. Clearly, the re-closing tear tab 8 could not adequately protect the packaging from damp or from entry of pollutants that would rapidly deteriorate any delicate product, such as fresh fruit and vegetables.

With reference to **Figures 3** and **4****,** a second example of a realization in the above field is given by the Japanese patent application JP 2006 290383 A that purports to solve the technical problem by packaging 20 which offers the possibility of either using a part of the bag as a cover once the bag itself has been opened or removing said part. In this solution the bag contains a number of disposable sanitary towels 21 packaged in a compressed form. The bag 20 comprises a bottom wall 22, a front wall 23, a rear wall 24 opposite the front wall 23, side walls 25 and 26 continuous with the front wall 23 and the rear wall 24, and an upper wall 27 opposite the bottom wall 22. A first perforated line 28 is made in the front wall 23 and in the side walls 25 and 26 substantially parallel to the direction of compression of the packaged products 21, and a second perforated line 29, of a tearing force superior to that of the first perforated line 28, is formed in the rear wall 24. On opening the packaging, the user can decide whether to tear the wall of the bag along the single perforated line 28 or along both lines 28 and 29. In the first case, that part of the packaging consisting of the upper wall 27, of the parts of front wall 23 and of lateral walls 25 and 26 in continuation with the upper wall 27, and of that part of rear wall 24 comprised between the upper wall 27 and the line of perforation 29, acts as a cover 30 that can be turned back along the perforating line 29. If, however, the user tears the wall along both perforated lines 28 and 29, the cover 30 is removed.

Although the presence of the fold-back cover 30 enables the bag 20 to be closed again after the required quantity of products 21 has been taken out, as a closing means it is not satisfactory as the edges of the cover 30 are simply laid close to the upper edges of the rest of the bag. Further, if the bag 20 is made of easily foldable material, such as a plastic film, when a few products have been taken out, the emptied part of both the bag and the cover will sink inward, allowing damp and various pollutants to enter and cause deterioration of the remaining products.

Added to all this, the presence of perforated lines 28 and 29 on the front wall 23, on the rear wall 24 and on both side walls 25 and 26, prevent the bag 20 from being used to enclose products compressed between the bottom wall 22 and the upper wall 27. In that case the products would in fact tend to cause premature tearing of the package.

### Purpose of the invention

Purpose of the present invention is to overcome the above drawbacks and to offer a packaging bag of flexible material, preferably film, to pack hygiene and other products, either compressed or uncompressed, able to protect the products from contact with pollutants once the bag has been opened and to preserve their quality.

### Summary of the invention

To achieve these purposes, subject of the invention is a packaging bag. preferably made of film material, for packaging products in general, said packaging bag having at least a wall in which at least one line prepared for tear-off is impressed to obtain an opening for access to the products, wherein:
- said at least a wall delimiting a volume for containing a quantity of products of any shape;
- said at least a wall carries a flap superimposed over the entire length of said tear-off line, the flap being connected to said wall by welding or by gluing along three of its sides in the guise of a pocket accessible from a remaining free side, entry of polluting substances through the above opening being prevented due to contact between the flap and the underlying wall, as described in claim 1.

Further characteristics of the present invention considered innovative are described in the dependent claims.

According to one aspect of the invention, a side of the flap is parallel to the tear-off line and is connected to the said wall in correspondence of matching edges of said wall.

According to one aspect of the invention, when in roll products are packaged, preferably hygiene products, said wall assumes a shape comprising a wall arbitrarily called a bottom wall, an upper wall opposite said bottom wall, a front wall, a rear wall opposite said front wall, two side walls continuous with said front wall and with said rear wall; said at least one tear-off line being impressed on said front wall continuous on both side walls without reaching the rear wall, and the flap being extended over contiguous portions of said upper, front, and side walls.

In one realised form the packaging bag also includes:
- an intermediate wall between said front wall and said rear wall, said intermediate wall being joined to said side walls, dividing said containing volume into two compartments;
- a second tear-off line impressed on said rear wall and continuing along both side walls;
- a second flap superimposed on said second tear-off line, the second flap being connected to said upper wall and to said side walls by welding or gluing along three of its sides in the guise of a pocket accessible from a remaining free side.

In one alternative realised form the packaging bag also includes:
- an intermediate wall between said side walls, said intermediate wall dividing said containing volume into two compartments;
- a second tear-off line impressed on said flap in alignment with the edge of said intermediate wall forming two separable parts one on each of the two compartments.

According to one aspect of the invention, one welded or glued side of the flap is connected to a band in which there is an approximately central opening to provide manual grip.

According to one aspect of the invention, the flap and said band for manual grip are made of the same material as that of the walls.

According to one aspect of the invention, the packaging bag includes reversible means for fixing the flap to the underlying wall on said free side of the flap.

From above it results that a peculiar characteristic of the packaging bag according to the present invention is that the flap superimposed on the opening, differently from the cover described in JP 2006 290383 A, or from the closure flaps of the common envelopes, cannot be turned back along a fold to allow the access to the content, but it must be moved away from the underlying wall, so determining a deformation.

Another subject of the invention is a manufacturing method for a packaging bag, the method including the steps of:
a) making a prepared tear-off line on a rectangular sheet, preferably made of film material, said line lying parallel to one side of said sheet;
b) superimposing a rectangular flap to said tear-off line over the entire length of the tear-off line,
c) folding on itself said sheet inclusive of the superimposed flap approximately to half length of a dimension of the sheet, along a folding line lying parallel to the tear-off line, obtaining two matching parts;
d) joining the edges of the two matching parts of the sheet and of flap, which edges are orthogonal to the folding line, by means of folding or gluing;
e) welding said two matching parts of the sheet inclusive of the flap along a welding line lying parallel to the tear-off line, said welding line either corresponding to the folding line or lying between the folding line and the tear-off line, in order to obtain a handle band in which there is an approximately central opening to provide manual grip, as described in the independent claim of method.

According to one aspect of the invention of method, a matching part exceeds the other by a margin of previously set width, in which two holes can be made for subsequent fork-lifting.

According to one aspect of the invention of method, said tear-off line is as long as said folding line.

According to one aspect of the invention of method, said flap exceeds said folding line and is welded or glued to the opposed matching part to reinforce said handle band.

According to one aspect of the invention of method, when the flap is ended on the folding line the handle band is welded or glued to one or the other matching parts near the welding line.

According to one aspect of the invention of method, said method also includes application of an adhesive strip across the free side of flap in contact with the underlying matching part, the length of said strip extending over part or all of the free side.

According to one aspect of the invention of method, said method also includes application of a double adhesive strip between flap and the underlying matching part in proximity to the free side of flap.

### Advantages of the invention

The present invention solves the problems mentioned in the known art described above. In particular, the shape of the flap in the guise of a pocket prevents entry of pollutants after the packaging bag has been opened; this means that said bag can be used for packaging food as well as hygiene products, ensuring that, once the bag is open, they will maintain unaltered their special qualities, aspect and characteristics like those of products that have only just been packaged.

Further, as the flap forms an integral part of the bag and is superimposed over the tear-off line, it prevents the products from falling out after the bag has been opened whatever the direction of compression may be.

Lastly, use of a flexible easily-folded material, typical of a plastic film, enables the packaging to adapt itself to the shape of the packaged product, within the limits imposed by its joins.

### Short description of the figures

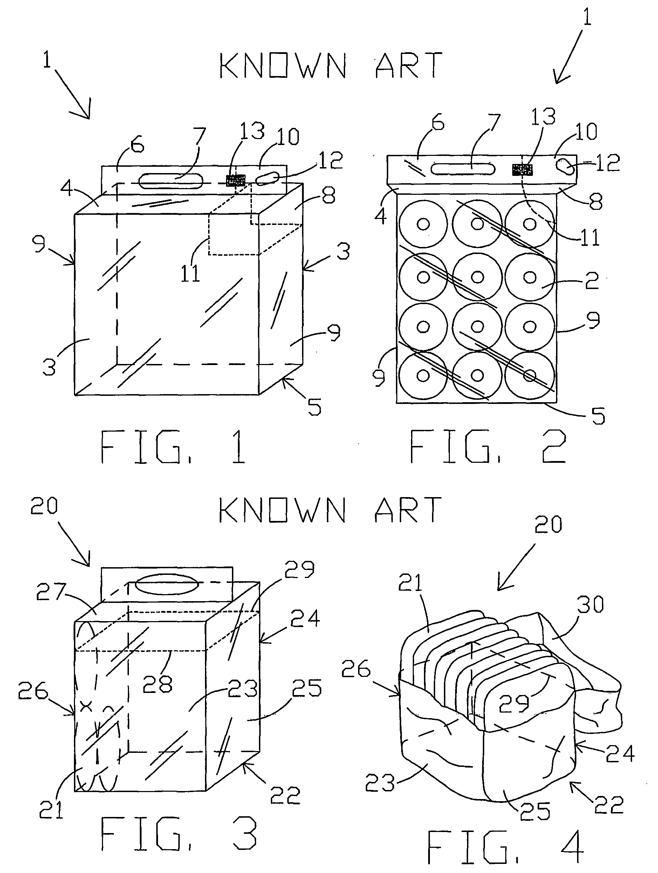

Further purposes and advantages of the present invention will be made clear by the following detailed description of an example of its realization, and by the attached drawings provided for purely explanatory purposes in no way limitative, wherein
**Figures 1** and **2** correspond to the Figures 1 and 6 of patent application WO 2008/104444 A1.
**Figures 3** and **4** correspond to the Figures 1a and 1b of patent application JP 2006 290383 A.
**Figure 5** shows a view in perspective of packed food where the packaging bag of the present invention has been used.
**Figure 6** shows the packaging bag in Figure 5 partly open along a prepared tear-off line.
**Figure 7** gives a plan view of a sheet and flap together, used in realizing a packaging bag according to the present invention.
**Figure 8** differs from Figure 7 in that the flap is narrower.
**Figure 9** gives a plan view of a packaging bag, obtained from the parts shown in Figure 7.
**Figures 10** to **13** are variants of the packaging bag in Figure 9.
**Figure 14** is a perspective in diagrammatic form of packaging of toilet paper rolls in a packaging bag as seen in Figures 7 to 13, fitted with a handle.
**Figure 15** shows the packaging bag in Figure 14 with the toilet paper rolls arranged differently.
**Figure 16** shows the packaging bag in Figure 14 with the flap raised after tearing the bag along the prepared tear-off line.
**Figure 17** shows the result of removing a roll from the packaging bag in Figure 16.
**Figures 18** to **23** show variants of the packaging bag in Figure 14.

### Detailed description of some preferred forms of realizing the invention

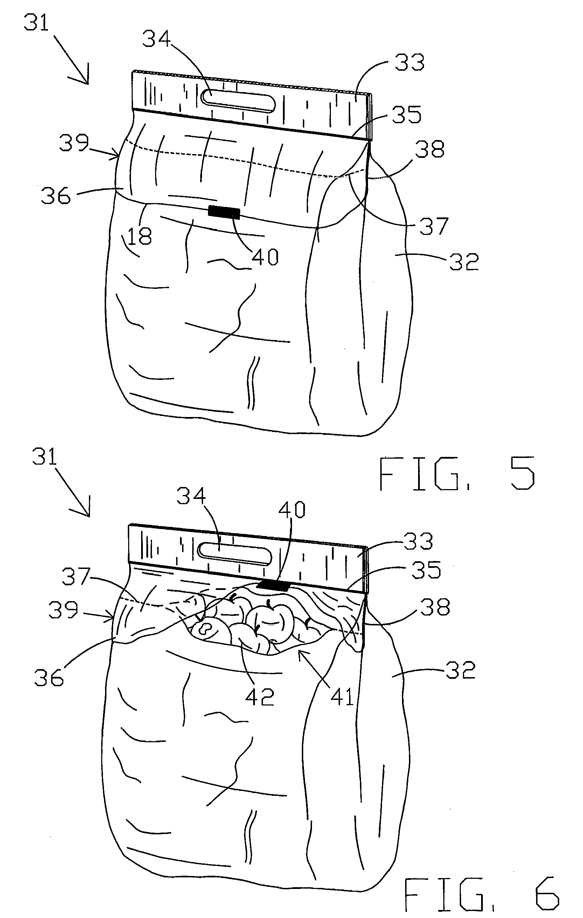

In the following description, the same parts that appear in different figures will be marked with the same symbols. When describing a figure, reference may be made to parts not shown in that figure but in preceding ones. Scales and proportions of the various parts shown are not necessarily the real ones. **Figure 5** shows a food packaging 31 one wall 32 of the packaging takes the form of a packaging bag when the products are packed inside it. A tear-off line 37 of suitable length is made on the wall 32. A flap 36 is superimposed over the tear-off line 37 for its whole length. A first side of the flap 36 is parallel to the tear-off line 37, said first side being connected to the wall 32 along a welding line 35. The flap 36 has two other opposite sides, 38 and 39, contiguous to the two ends of the first side, and a fourth side 18 opposite the first side 35. Sides 38 and 39 are also joined to the wall 32, keeping the flap 36 anchored to the underlying wall 32 around the contour, except for side 18, opposite the welding line 35 which is left free, namely without a welding. A hand can therefore be inserted into the reversed pocket, formed by the flap 36 with the underlying wall 32, to tear the packaging bag at the tear-off line 37 and gain access to the products. Tear-off line 37 is made by perforations suitably spaced according to the tearing force to be applied to it. Whenever a product is taken from the bag, an adhesive strip 40, fixed on the free side 18, keeps flap 36 adhering to wall 32 to ensure that the remaining products maintain their high level of quality.

Where present, a rectangular band 33, in which there is a long central opening 34 for manual grip and for lifting the food packaging 31, is also welded to welding line 35 along its entire length. The wall 32, flap 36 and band 33 are all made of the same polyethylene film, more generally of polyolefin. This in no way limits the possibility of using other materials possessing the same flexibility, or of using different materials for the three parts 32, 33 and 36. **Figure 6** shows the food packaging 31 in Figure 5 wherein the flap 36 is partially detached from the wall 32 of the packaging bag, showing, through a tear 41 of the wall 32 along a section of the tear-off line 37, some of the apples 42 contained in the homonym bag 32. One or more apples 42 can be taken out of said food packaging 31. Having done so, the two edges of the tear 41 can be covered by the flap 36 which is then fixed to the wall 32 by the adhesive strip 40 (a position not shown in the figure). The adhesive applied to the strip 40 is sensitive to pressure.

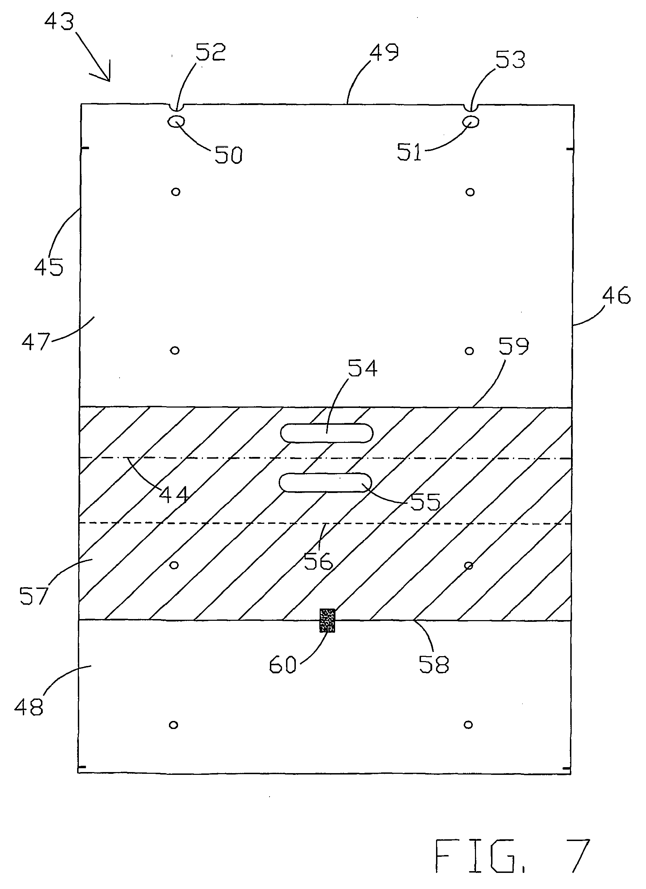

**Figure 7** shows a rectangular sheet 43 made of the material already mentioned for the food packaging 31. On the surface of sheet 43 in the figure is a crease line 44 extending crosswise from one longitudinal side 45 to the other 46 at a certain distance from the crosswise axis of the sheet 43, dividing said sheet into two parts, 47 and 48, of different lengths. Two holes for fork-lifting, 50 and 51, are made in one shorter side 49 of sheet 43. On side 49, two semicircular cuts, 52 and 53, to facilitate tearing by fork-lifting, are made close to said holes 50 and 51. Two elongated crosswise holes, 54 and 55, are made in a central position symmetrically to the crease line 44. On section 48 of sheet 43, beyond the hole 55 in relation to crease line 44, a tear-off line 56, parallel to crease line 44, extends from side 45 to side 46 of sheet 43, but this line could even be shorter. A second sheet 57, as wide as sheet 43 but much shorter, is superimposed over this latter so as to cover a central part containing the two elongated holes 54 and 55, the crease line 44 and a section of suitable width beyond the tear-off line 56. The longer edges, 58 and 59, of the second sheet 57, parallel to the crease line 44, lie at different distances from said crease line or, stated more precisely, edge 58 lies at the greater distance beyond the tear-off line 56. The material of second sheet 57 is the same as that of sheet 43 but could be of another material of comparable flexibility. In the second sheet 57 are two elongated holes that, in the superimposition shown in the figure, coincide with elongated holes 54 and 55. An adhesive tongue 60 is placed in a central position across the edge 58 of sheet 57. The adhesive used on the tongue 60 is pressure sensitive.

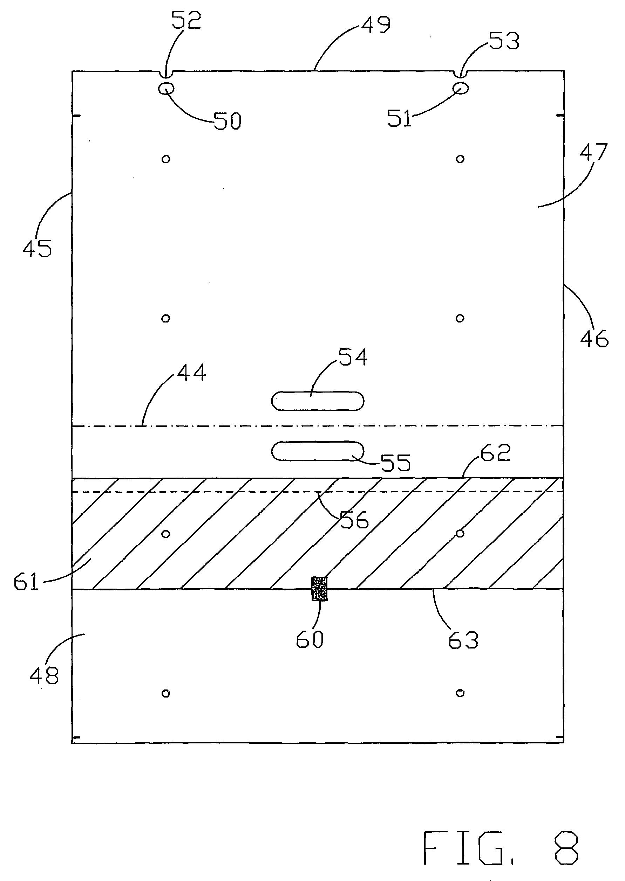

**Figure 8** differs from Figure 7 in that it presents a second rectangular sheet 61 narrower than sheet 57. Sheet 61 is superimposed only over part 48 of sheet 43. Sheet 61 is across the tear-off line 56 and with a longer side 62 comprised between elongated hole 55 and tear-off line 56. Placed in a central position across the other longer side 63 of sheet 61 is the adhesive tongue 60.

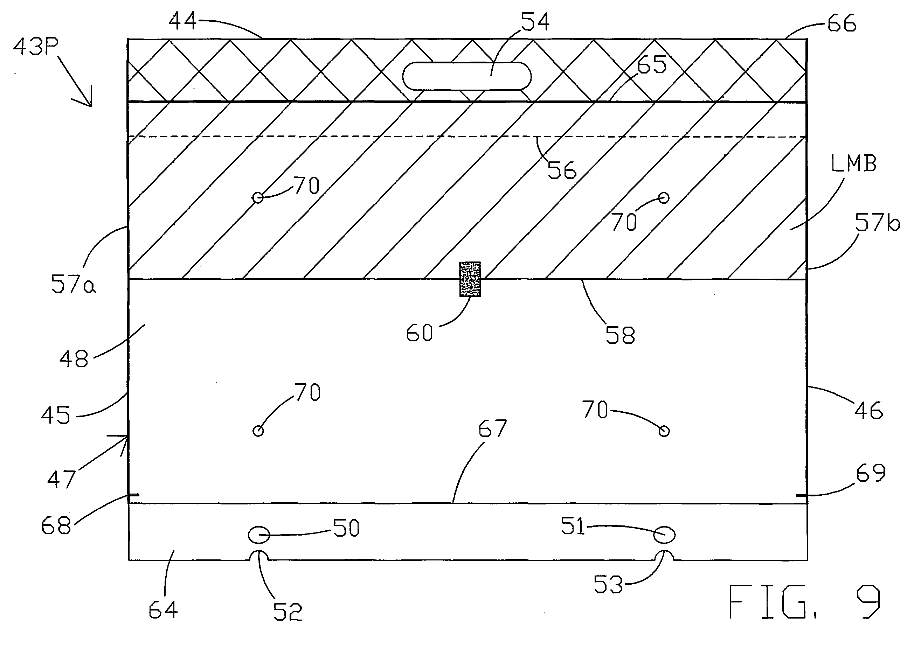

**Figure 9** shows the rectangular sheet 43 bent back onto itself at crease line 44 so that parts 47 and 48 of sheet 43 match except for a margin 64 that contains the fork-lifting holes 50 and 51. The second sheet 57 is also folded along crease line 44 so as to match with parts 47 and 48 of sheet 43. The two matching parts 47 and 48 of sheet 43 and the two parts of sheet 57 that match with both, are welded along the common edges perpendicular to crease 44. A welding 65 parallel to crease 44 transversally joins together the parts previously welded along the edges. The distance between welding line 65 and crease 44 is less than that between this latter and the tear-off line 56, so that a longitudinal band 66 is formed between crease 44 and welding line 65, said band containing the elongated holes 54 and 55 aligned to form an opening for manual grip. This creates a flap LMB delimited by the welding line 65, by the longitudinal free (unwelded) edge 58 of sheet 57 and by the two sections 57a, 57b of the joined edges 45, 46 comprised between the welding line 65 and the free edge 58.

The assembly described above can function as a packaging bag 43P for packaging any kind of product, such as those for hygiene use or for food. During production, these packaging bags are piled up onto the bars of the rack after being fork-lifted through holes 50 and 51. Electro-mechanical systems pick up single packaging bags from the pile by tearing through margin 64 at the semicircular cuts 52, 53, and separate the two parts 47 and 48 of sheet 43 on the free edge 67 so that the products to be packaged can by put in. Two cuts, 68 and 69, are made in the sheet 43 across the joined edges 45 and 46 in proximity to the margin 64 in order to absorb the shock caused by the electro-mechanical system when opening the packaging bag and subsequently filling it. When the packaging bag 43P has been filled with the desired quantity of a product, the two parts 47 and 48 of sheet 43, previously welded along the edges 45 and 46, are welded together at the margin 64 thus closing the packaging bag 43P. At a suitable position on each of the two parts 47 and 48 of sheet 43, cuts 79 are made without removal of any material, their function being to act as vents allowing any remaining air to escape from the package once it has been closed.

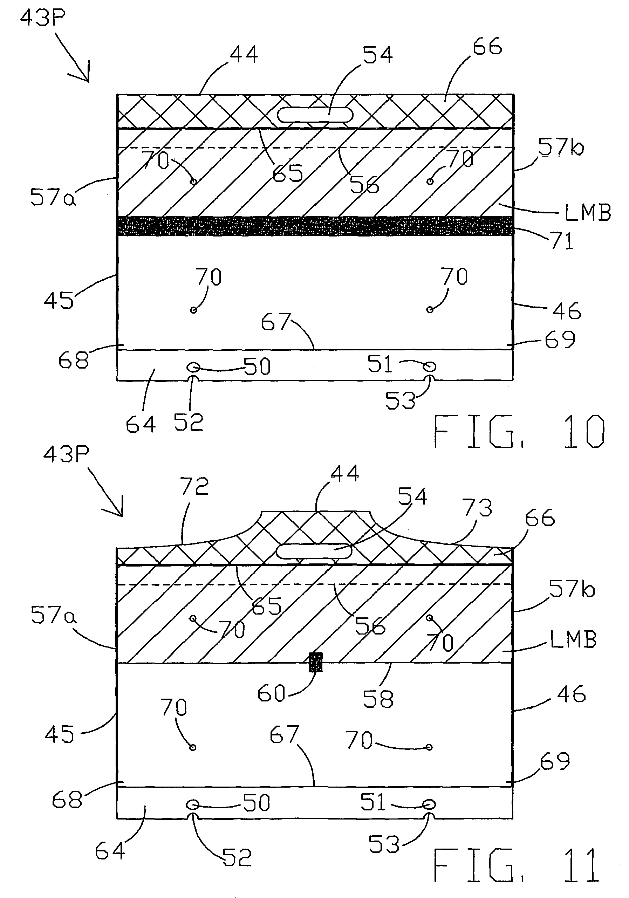

**Figure 10** differs from Figure 9 in that the adhesive tongue 60 is replaced by an adhesive strip 71 extending across edge 58 along its entire length.

Alternatively a double adhesive strip (not shown in the figure) can be applied between the flap LMB and the underlying part 48 of sheet 43. In both cases the strip can be shorter than the edge 58.

**Figure 11** differs from Figure 9 in that there are two curved cuts, 72 and 73, in the band forming the handle 66. Starting from the two respective joined edges 45 and 46, said cuts, in proximity to the welding line 65, converge towards a central segment of the crease 44 above the opening 54 (and 55) to provide manual grip.

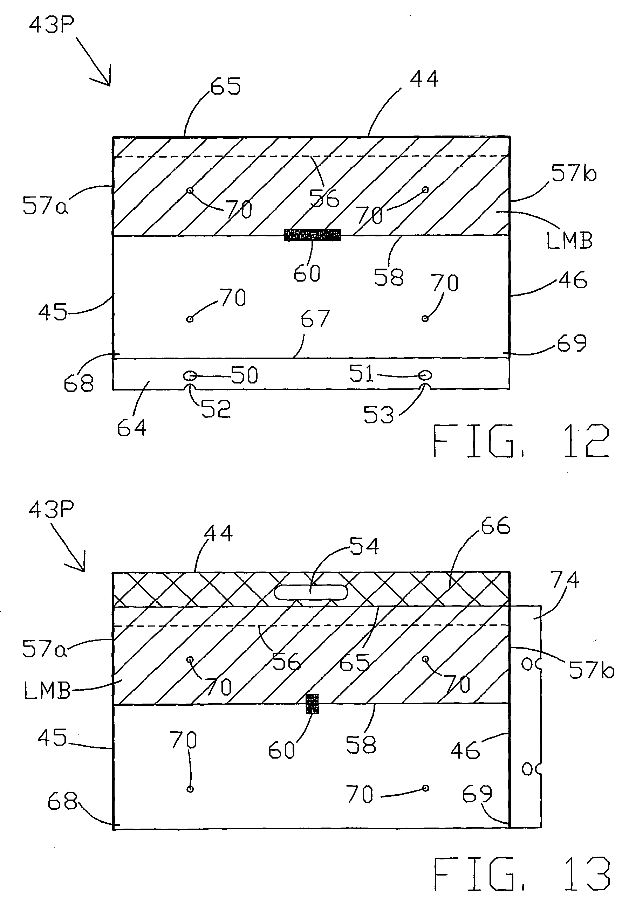

**Figure 12** differs from Figure 9 in that the packaging bag 43P has no band for a handle 66.

**Figure 13** differs from Figure 9 in that the packaging bag 43P lacks the margin 64 for fork-lifting, there being another margin 74 for this purpose that extends laterally beyond the welded edge 46 of packaging bag 43P.

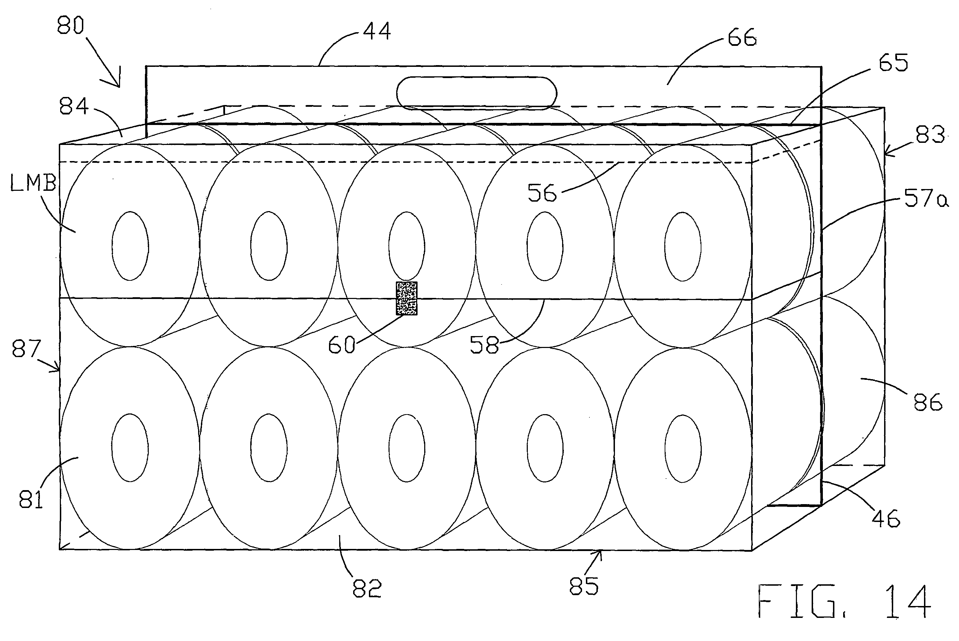

**Figure 14** shows a packaging 80 for two superimposed rows of toilet paper rolls 81 enclosed in the packaging bag 43P in Figure 9, in which the tear-off line 56 is still intact and the flap LMB is held firmly in place by the adhesive tongue 60. Due to these characteristics: the film material of which it is made, the three weldings 45, 46 and 65 perpendicular one to another, and the fourth welding made after filling has been completed, the packaging bag 43P surrounds and adapts itself with rounded edge to the substantially parallelepiped shape of the contents. In this way the following walls are created: a front wall 82 that comprises the greater length of flap LMB; a rear wall 83 opposite the front wall 82; an upper wall 84 to the middle of which the band of handle 66 is connected at the welding line 65; a bottom wall 85 opposite the upper wall 84; two side walls 86 and 87 continuous with the front wall 82 and with the rear wall 83. The tear-off line 56 is parallel to the upper wall 84 and extends over the whole length of the front wall 82 in proximity to the upper wall 84, continuing always parallelly to the upper wall 84 over both side walls 86 and 87 up to half their width. Starting from the welding line 65, the flap LMB covers half the upper wall 84 continuing beyond the tear-off line 56 over part of the front wall 82 and onto part of the two side walls 86 and 87 for half their width. Flap LMB is welded, at its shorter sides 57a and 57b, to the side walls 86 and 87 along the joined edges 45 and 46 of the packaging bag 43P. The free edge 58 of the flap LMB also extends onto the side walls 86 and 87. The axis of the rolls 81 lies perpendicular to the front wall 82.

The weldings 57a and 57b and the welding 65 perpendicular to the weldings 57a and 57b, keep the flap LMB fixed to the packaging bag 43P over three sides, like a pocket turned upside down. It follows that flap LMB, contrary to the flaps on ordinary envelopes, cannot turn around a longitudinal crease to make the opening in the bag accessible, but must be moved from the underlying front wall 82 manually, causing deformation both of the flap LMB itself and of the packaging bag 43P.

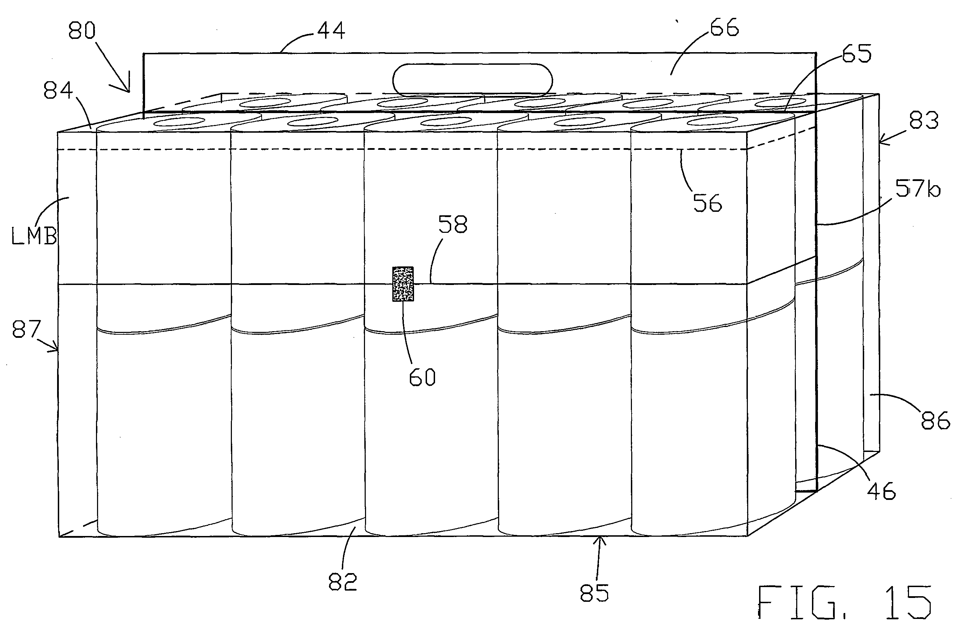

**Figure 15** differs from Figure 14 in that the rolls are differently arranged inside the package 80. The axis of the rolls, in particular, is perpendicular to the upper wall 84.

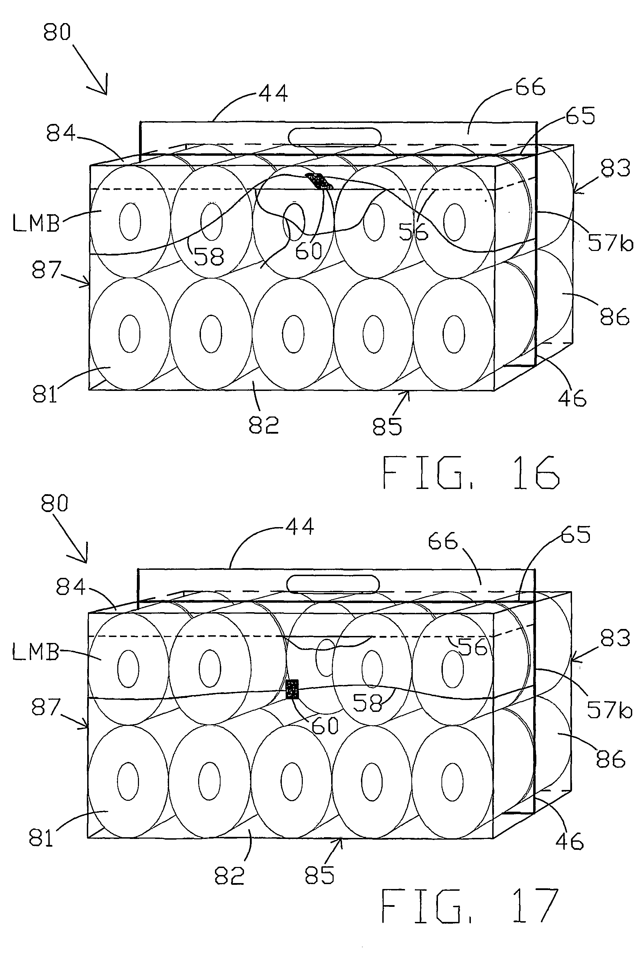

**Figure 16** shows the package 80 wherein the tongue 60 is detached from the front wall 82, the flap LMB has been manually moved from the central part of said wall and the packaging bag 43P is torn along the tear-off line 56 below the part of the flap LMB that has been moved, so that a roll 81 of toilet paper can be taken out.

Figure 17 shows the package 80 in Figure 16 after a roll 81 has been removed, the previously detached flap LMB has sunk back against the front wall 82 and held in place by application of slight pressure on the adhesive tongue 60.

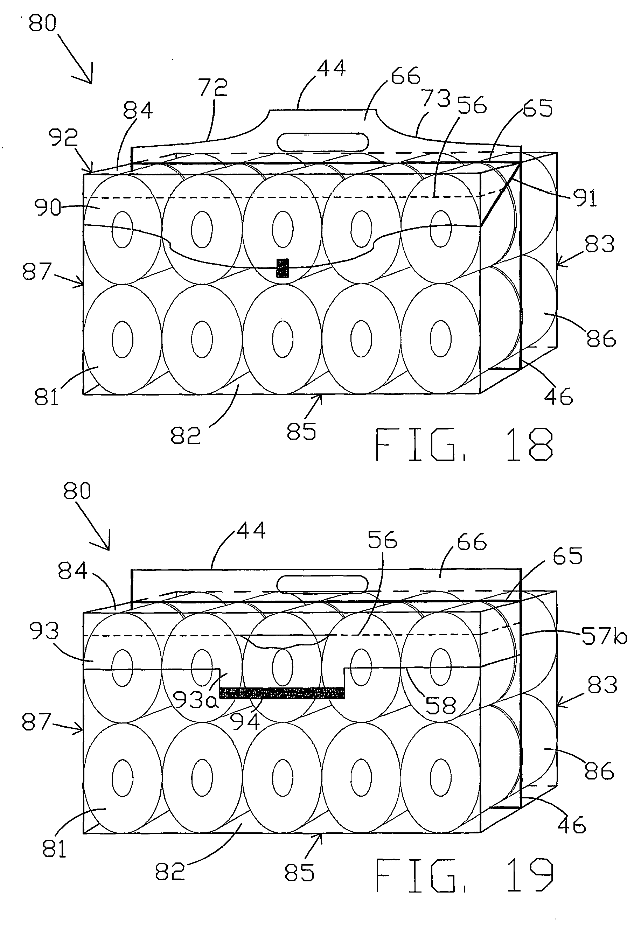

In **Figure 18** the band of handle 66 is shaped as shown in Figure 11 while the flap 90 differs from flap LMB in that its free edge is superimposed only over the front wall 82, in a symmetrically curving profile in relation to a crosswise axis. The flap 90 is joined to each side wall 86 and 87 along an oblique welding line, respectively 91 and 92, which runs from the end of welding line 65 to the end of the free edge of flap 90.

In **Figure 19** the flap 93 differs from the flap LMB in that the wall superimposed over the front wall 82 presents a substantially rectangular widening 93a in its central part, including an adhesive strip 94.

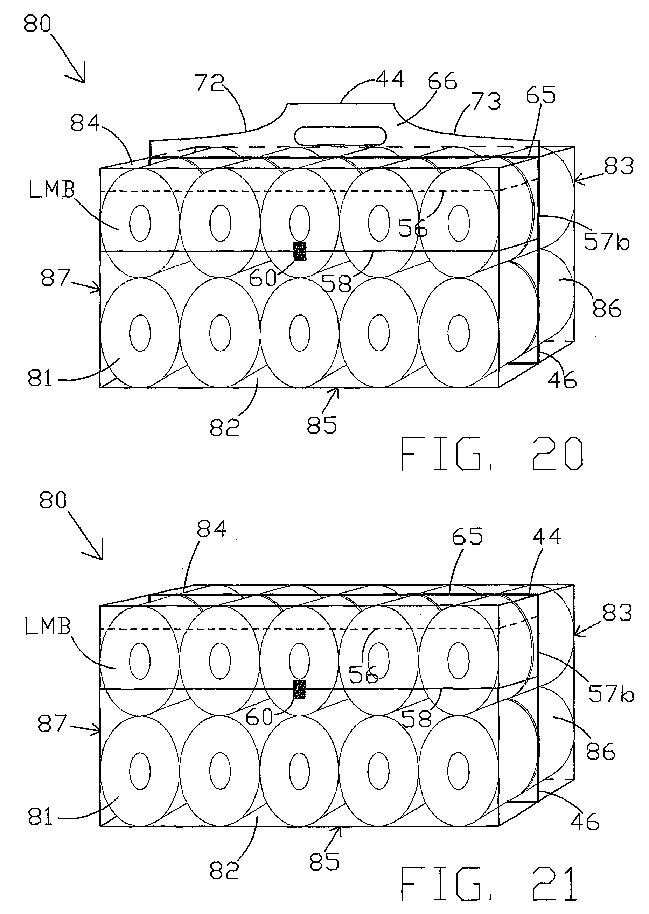

**Figures 20** and **21** show the packages obtained by filling the packaging bags illustrated in Figures 11 and 12.

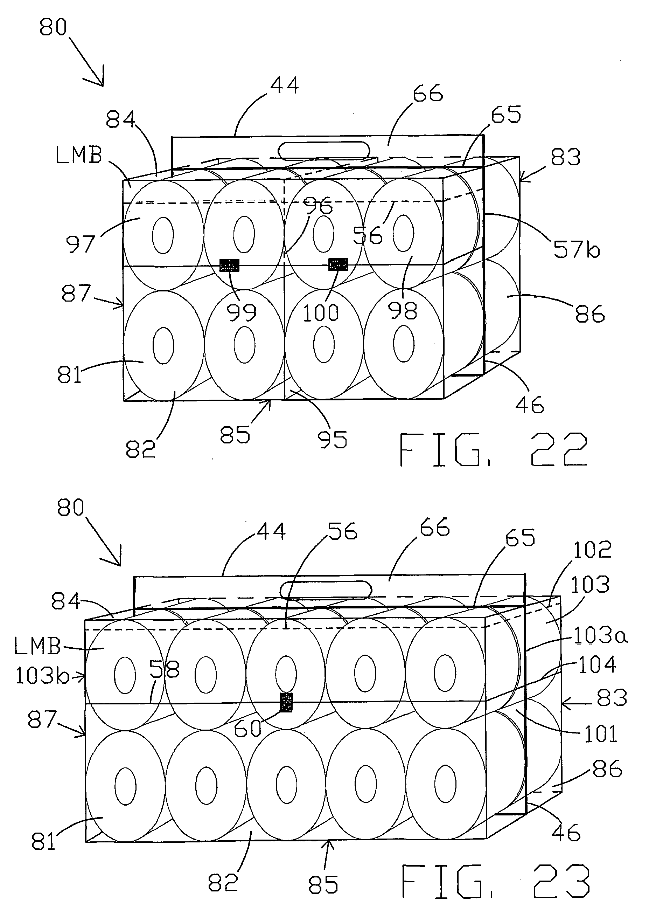

**Figure 22** differs from Figure 14 in that it shows the presence of an intermediate wall 95 between side walls 86 and 87, in a central position parallel to both, that divides the containing volume into two compartments. A crosswise tear-off line 96 aligned to the edge of intermediate wall 95 is impressed on flap LMB. The tear-off line 96 divides flap LMB into two parts 97 and 98, one for each compartment, each part having its adhesive tongue, 99 and 100, to the front wall 82 in correspondence of the free side of the flap LMB. In this way a roll can be taken from either of the two compartments into which the packaging bag 43P is divided. The band of handle 66 extends over both compartments.

One method of manufacturing the packaging bag in Figure 22 presumes insertion of a third sheet between the two superimposed parts, 47 and 48, of the sheet 43 in Figure 9, before the welding is made along the edges 45 and 46. When the first rolls of toilet paper 81 are put into the packaging bag 43P, balancing from one and the other ends of the opening in correspondence of the free side 67, the two compartments are formed, divided by the intermediate wall 95, produced by stretching exerted by reciprocal movement of the superimposed parts 47 and 48, that give rise to the parallelepiped shape of the packaging bag 43P.

**Figure 23** differs from Figure 14 by the presence of an intermediate wall 101 between the front wall 82 and the rear wall 83, parallel to both in a central position, that divides the containing volume into two compartments. A second tear-off line 102 is also made, parallel to the upper wall 84, on the rear wall 83 for its whole length, continuing on both side walls 86 and 87 till it meets the joined edges 45 and 46. Starting from the welding line 65, a second flap 103 covers the other half of the upper wall 84 continuing beyond tear-off line 102 on the rear wall 83 and on the two side walls 86 and 87 for half their width. The flap 103 is welded at its shorter sides 103a, 103b to side walls 86 and 87 along the joined edges 45 and 46 of packaging bag 43P. The free edge 104 of flap 103 also extends over side walls 86 and 87.

A method of manufacturing the packaging in Figure 23 presumes insertion of a third sheet between the two superimposed parts 47 and 48 of sheet 43, in Figure 9, before the edges 45 and 46 are welded. Width of the third sheet is the same as that of sheet 43 its length being little less than the distance between the free edge of part 48 and welding line 65. The third sheet is fitted in between the two superimposed parts 47 and 48 the crosswise margins being respectively aligned with edges 45 and 46 of sheet 43. The weldings along edges 45 and 46 unite the third sheet to the two superimposed parts 47 and 48. On inserting the first rolls of toilet paper 81 in the packaging bag 43P, balancing from one and the other edges of the opening in correspondence of the free side 67, two compartments are formed separated by the intermediate wall 101, created by stretching exerted by the reciprocal movement of superimposed parts 47 and 48 that give rise to the parallelepiped form of packaging bag 43P.

Based on the description given of a preferred example of realization of the invention, some changes can clearly be made by experts in the field without thereby departing from the sphere of the invention as will be explained in the following claims.

## Claims

1. A flexible packaging bag, preferably made of film material, for packaging products in general, said packaging bag having at least a wall (32) in which at least one line prepared for tear-off (37) is impressed to obtain an opening (41) for access to the products (42),
- said at least a wall (32) delimiting a volume for containing a quantity of products of any shape;
- said at least a wall (32) carries a flap (36) superimposed over the entire length of said tear-off line (37), the flap (36) being connected to said wall (32) by welding or by gluing along three of its sides (35, 38, 39) in the guise of a pocket accessible from a remaining free side (18), entry of polluting substances through the above opening (41) bein prevented due to contact between the flap (36) and the underlying wall (32); **characterised in that**: one welded or glued side of the flap (36) is connected to a band (33, 66) in which there is an approximately central opening (34) to provide manual grip.

2. Packaging bag as in claim 1, **characterised in that**, a side (35) of the flap (36) is parallel to the tear-off line (37) and is connected to the said wall (32) in correspondence of matching edges of said wall.

3. Packaging bag (43P) as in claim 1, **characterised in that**, when in-roll products are packaged, preferably hygiene products, said wall assumes a shape comprising a wall arbitrarily called a bottom wall (85), an upper wall (84) opposite said bottom wall (85), a front wall (82), a rear wall (83) opposite said front wall (82), two side walls (86, 87) continuous with said front wall (82) and with said rear wall (83); said at least one tear-off line (56) being impressed on said front wall (82) continuous on both side walls (86, 87) without reaching the rear wall (83), and the flap (LMB) being extended over contiguous portions of said upper (84), front (82), and side walls (86, 87).

4. Packaging bag (43P) as in claim 3, **characterised in that** it includes:
- an intermediate wall (101) between said front wall (82) and said rear wall (83), said intermediate wall being joined to said side walls (86, 87), dividing said containing volume into two compartments;
- a second tear-off line (102) impressed on said rear wall (83) and continuing along both side walls (86, 87);
- a second flap (103) superimposed on said second tear-off line (102), the second flap (103) being connected to said upper wall (84) and to said side walls (86, 87) by welding or gluing along three of its sides (65, 103a, 103b) in the guise of a pocket accessible from a remaining free side (104).

5. Packaging bag (43P) as in claim 3, **characterised in that** it includes:
- an intermediate wall (95) between said side walls (86, 87), said intermediate wall (95) dividing said containing volume into two compartments;
- a second tear-off line (96) impressed on said flap (LMB) in alignment with the edge of said intermediate wall (95) forming two separable parts (97, 98) one on each of the two compartments.

6. Packaging bag (32, 43P) as in claim 1, **characterised in that** the flap (36, LMB) and said band (33) for manual grip are made of the same material as that of the walls.

7. Packaging bag (32, 43P) as in any one of the preceding claims, **characterised in that** it includes reversible means (40) for fixing the flap (36) to the underlying wall (32) on said free side (18) of the flap (36).

8. Method for manufacturing the packaging bag as in claim 1, including the steps of:
a) making a prepared tear-off line (56) on a rectangular sheet (43), preferably made of film material, said line (56) lying parallel to one side (49) of said sheet (43);
b) superimposing a rectangular flap (57, 61) to said tear-off line (56) over the entire length of the tear-off line (56),
c) folding on itself said sheet (43) inclusive of the superimposed flap (LMB) approximately to half length of a dimension of the sheet, along a folding line (44) lying parallel to the tear-off line (56), obtaining two matching parts (47, 48);
d) joining the edges (45, 46) of the two matching parts (47, 48) of the sheet (43) and of flap (LMB), which edges are orthogonal to the folding line (44), by means of folding or gluing;
e) welding said two matching parts (47, 48) of the sheet (43) inclusive of the flap (LMB) along a welding line (65) lying parallel to the tear-off line (56), said welding line (65) either corresponding to the folding line (44) or lying between the folding line (44) and the tear-off line (56), in order to obtain a handle band (66) in which there is an approximately central opening (54, 55) to provide manual grip.

9. Method as in claim 8, **characterised in that** a matching part (47) exceeds the other (48) by a margin (64) of previously set width, in which two holes (50, 51) can be made for subsequent fork-lifting.

10. Method as in claim 8, **characterised in that** said tear-off line (56) is as long as said folding line (44).

11. Method as in claim 8, **characterised in that** said flap (LMB) exceeds said folding line (44) and is welded or glued to the opposed matching part (47) to reinforce said handle band (66).

12. Method as in claim 8, **characterised in that** when the flap (57) is ended on the folding line (44) the handle band (66) is welded or glued to one or the other matching parts (47, 48) near the welding line (65).

13. Method as in claim 8, **characterised in that** it also includes application of an adhesive strip (60) across the free side (58) of flap (LMB) in contact with the underlying matching part (48), the length of said strip (60) extending over part or all of the free side (58).

14. Method as in claim 8, **characterized in that** it also includes application of a double adhesive strip between flap (LMB) and the underlying matching part (48) in proximity to the free side (58) of flap (LMB).

## Patentansprüche

1. Flexibles Verpackungsbeutel, vorzugsweise aus einem Material wie einem Film hergestellt, im Allgemeinen zum Verpacken von Produkten, wobei das besagte Verpackungsbeutel mindestens eine Wand (32) aufweist, in der wenigstens eine vorbereitete Reißöffnungslinie (37) graviert ist, um eine Öffnung (41) für einen Zugang zu den Produkten (42) zu erhalten, wobei
- mindestens eine Wand (32) begrenzt ein Volumen zur Aufnahme einer Menge von Produkten in beliebiger Form;
- mindestens eine Wand (32) aufweist einen Flügel (36), der über die gesamte Länge auf der besagten Reißöffnungslinie (37) überlagert ist, wobei der Flügel (36) zu der genannten Wand (32) durch Schweißen oder Kleben, entlang drei von seiner Seiten (35, 38, 39) in der Art einer von einer frei gebliebenen Seite (18) zugänglichen Tasche, verbunden ist, wobei der Eintritts von Verunreinigungen durch die oben genannten Öffnung (41) durch den Kontakt zwischen dem Flügel (36) und der unterliegenden Wand (32) verhindert wird, **dadurch gekennzeichnet, dass** eine geschweißte oder verklebte Seite des Flügels (36) mit einem Band (33, 66) verbunden ist, wobei eine ungefähr zentrale Öffnung (34) vorhanden ist, um einen manuellen Griff zu erreichen.

2. Verpackungsbeutel nach Anspruch 1, **dadurch gekennzeichnet, dass** eine Seite (35) des Flügels (36) parallel zur Reißöffnungslinie (37) und mit der besagten Wand (32) bei gekoppelten Kanten der besagten Wand, verbunden ist.

3. Verpackungsbeutel (43P) nach Anspruch 1, **dadurch gekennzeichnet dass**, wenn die Produkte, vorzugsweise Hygieneprodukte in Rollen verpackt werden, hat die besagte Wand eine solche Gestalt, die eine Wand umfasst, die willkürlich als Bodenwand (85), der Bodenwand (85) abgewandte obere Wand (84), Vorderwand (82), der besagten Vorderwand (82) abgewandte Rückwand (83), zwei Seitenwände (86, 87), die kontinuierlich mit der besagten Vorderwand (82) und mit der besagten Rückwand (83) sind, genannt wird; wobei zumindest eine Reißöffnungslinie (56) auf der besagten Frontwand (82) in kontinuierlicher Weise an beiden Seitenwänden (86, 87) graviert ist, ohne die Rückwand (83) zu erreichen, und der Flügel (LMB) sich über die angrenzenden Teile der besagten oberen (84), vorderen (82) und seitlichen Wänden (86, 87) entwickelt.

4. Verpackungsbeutel (43P) nach Anspruch 3, **dadurch gekennzeichnet dass** es folgendes umfasst:
- eine Zwischenwand (101) zwischen der besagten Vorderwand (82) und der besagten Rückwand (83), wobei die besagte Zwischenwand mit den besagten Seitenwänden (86, 87) verbunden und das besagte Aufnahmevolumen in zwei Abteilungen verteilt;
- eine zweite Reißöffnungslinie (102) die auf der besagten Rückwand (83) graviert und weiter entlang beider Seitenwände (86, 87) fortsetzt;
- einen zweiten auf der besagten zweiten Reißöffnungslinie (102) überlagerten Flügel (103), wobei der zweite Flügel (103) mit der oberen Wand (84) und den besagten Seitenwänden (86, 87) durch Schweißen oder Kleben, entlang drei von seinen Seiten (65, 103a, 103b), in der Art einer von einer frei gebliebenen Seite (104) zugänglichen Tasche, verbunden wird.

5. Verpackungsbeutel (43P) nach Anspruch 3, **dadurch gekennzeichnet, dass** es folgendes umfasst:
- einer Zwischenwand (95) zwischen den besagten Seitenwänden (86, 87), wobei die besagte Zwischenwand (95) das besagte Rückhaltevolumen in zwei Abteilungen verteilt;
- eine zweite auf dem besagten Flügel (LMB) in Ausrichtung mit der Kante der besagten Zwischenwand (95) gravierte Reißöffnungslinie (96), die zwei trennbare Teile (97, 98), ein auf jeder der beiden Abteilungen, bildet.

6. Verpackungsbeutel (32, 43P) nach Anspruch 1, **dadurch gekennzeichnet, dass** der Flügel (36, LMB) und der besagte Band (33) aus dem gleichen Material der Wände gebildet sind, um einen Handgriff zu erreichen.

7. Verpackungsbeutel (32, 43P) nach einem der vorhergehenden Ansprüche, **dadurch gekennzeichnet, dass** es reversible Mittel (40) aufweist, um den Flügel (36) an der unteren Wand (32) auf der besagten freien Seite (18) des Flügels (36) zu sichern.

8. Verfahren zur Herstellung des Verpackungsbeutels nach Anspruch 1, folgende Schritte umfassend:
a) eine vorbereitete Reißöffnungslinie (56) auf einer rechteckigen Folie (43) ausbilden, die vorzugsweise aus einem Material in Form eines dünnen Films ausgebildet ist, wobei die besagte Linie (56) parallel zu einer Seite (49) der besagten Folie (43) ist;
b) einen rechteckigen Flügel (57, 61) auf der besagten Reißöffnungslinie (56) für die gesamte Länge der Reißöffnungslinie (56) überlagern,
c) die besagte Folie (43) inklusive des übergelagerten Flügels (LMB) etwa auf halber Länge einer Abmessung der Folie entlang einer Faltlinie (44) parallel zur Reißöffnungslinie (56) falten, wobei zwei Kupplungsteilen (47, 48) erhalten werden;
d) die Ränder (45, 46) der beiden Kupplungsteile (47, 48) der Folie (43) und des Flügels (LMB) verbinden, wobei die Ränder senkrecht zur Faltlinie (44) sind, mittels Biegen oder Kleben;
e) die beiden Kupplungsteile (47, 48) der Folie (43), einschließlich des Flügels (LMB) entlang einer parallel zur Reißöffnungslinie (56) angeordneten Schweißlinie (65) verbinden, wobei die besagte Schweißlinie (65) entweder der Faltlinie (44) entspricht, oder zwischen der Faltlinie (44) oder der Reißöffnungslinie (56) liegt, um einen Band (66) mit einem manuellen Griff zu erhalten, in welchem eine Öffnung (54, 55) etwa mittig liegt, um den Handgriff zu erreichen.

9. Verfahren nach Anspruch 8, **dadurch gekennzeichnet, dass** ein Kupplungsteil (47) das andere (48) entsprechend einer Marge (64) mit einer Länge R übersteigt, die zuvor festgelegt wird, wobei zwei Löcher (50, 51) für einen nachfolgenden anheben mittels einer Gabel hergestellt werden können.

10. Verfahren nach Anspruch 8, **dadurch gekennzeichnet, dass** die besagte Reißöffnungslinie (56) die gleiche Länge der besagten Faltlinie (44) aufweist.

11. Verfahren nach Anspruch 8, **dadurch gekennzeichnet, dass** der besagte Flügel (LMB) die besagte Faltlinie (44) übersteigt und geschweißt oder verklebt auf dem gegenüberstehende Kupplungsteil (47) ist, um das besagte Band (66) des Handgriffes zu verstärken.

12. Ein Verfahren nach Anspruch 8, **dadurch gekennzeichnet, dass**, wenn der Flügel (57) an der Faltlinie (44) endet, wird das Band (66) des Handgriffes auf einem oder anderen der Kupplungsteile (47, 48) neben der Schweißlinie (65) geschweißt oder verklebt.

13. Verfahren nach Anspruch 8, **dadurch gekennzeichnet, dass** es auch das Aufbringen eines Klebestreifens (60) durch die freie Seite (58) des Flügels (LMB) in Kontakt mit dem unterliegenden Kupplungsteil (48) umfasst, wobei sich die Länge des besagten Streifens (60) auf einem Teil oder auf der gesamten freien Seite (58) erstreckt.

14. Verfahren nach Anspruch 8, **dadurch gekennzeichnet, dass** es er ferner das Aufbringen eines Doppelklebestreifen zwischen dem Flügel (LMB) und dem unterliegenden Kupplungsteil (48), in der Nähe der freien Seite (58) des Flügels (LMB), umfasst.

## Revendications

1. Sac flexible d'emballage, de préférence formé à partir d'un matériau d'un film mince, pour emballer des produits en général, ledit sac d'emballage ayant au moins une paroi (32) dans laquelle est gravée au moins une ligne préparée pour une ouverture à déchirer (37), afin d'obtenir une ouverture (41) pour un accès aux produits (42),
- ladite paroi au moins unique (32) délimite un volume destiné à contenir un quantité de produits de toute forme;
- ladite paroi au moins unique (32) porte un volet (36) superposée sur toute la longueur de ladite ligne d'ouverture à déchirer (37), le volet (36) étant lié à ladite paroi (32) par soudage ou collage le long de trois de ses côtés (35, 38, 39), à la manière d'une poche accessible depuis un côté (18) est resté libre, ce qui empêche l'entrée des polluants dans l'ouverture précitée (41) en raison d'un contact entre le volet (36) et la paroi (32) sous-jacente, **caractérisé en ce qu'**un côté (36) soudé ou collé est lié à un ruban (33, 66) dans lequel se trouve une ouverture (34) presque centrale, à fin de réaliser une préhension manuelle.

2. Sac d'emballage selon la revendication 1, **caractérisé en ce qu'**un côté (35) du volet (36) est parallèle à la ligne d'ouverture à déchirer (37) et est relié à ladite paroi (32) au niveau des bords couplés de ladite paroi.

3. Sac d'emballage (43P) selon la revendication 1, **caractérisé en ce que**, lorsque des produits sont emballés en rouleaux, de préférence des produits d'hygiène, ladite paroi a une forme comportant une paroi arbitrairement appelée paroi de fond (85), une paroi supérieure (84) opposée à ladite paroi de fond (85), une paroi avant (82), une paroi arrière (83) opposée à ladite paroi avant (82), deux parois latérales (86, 87) en continu avec ladite paroi avant (82) et avec ladite paroi arrière (83); ladite au moins unique ligne d'ouverture à déchirer (56) étant gravée sur ladite paroi avant (82) en continu sur les deux parois latérales (86, 87) sans atteindre la paroi arrière (83), et le volet (LMB) s'étendant au-dessus de parties contiguës desdites parois supérieure (84), avant (82) et latérale (86, 87).

4. Sac d'emballage (43P) selon la revendication 3, **caractérisé en ce, qu'**il comprend:
- une paroi intermédiaire (101) entre ladite paroi avant (82) et ladite paroi arrière (83), ladite paroi intermédiaire étant reliée aux dites parois latérales (86, 87), en séparant ledit volume de confinement en deux compartiments:
- une deuxième ligne d'ouverture détachable (102) gravée sur ladite paroi arrière (83) et en continuant le long des deux parois latérales (86, 87);
- un deuxième volet (103) superposé sur ladite deuxième ligne d'ouverture détachable (102), le deuxième volet (103) étant relié à ladite paroi supérieure (84) et auxdites parois latérales (86, 87) par soudage ou collage le long de trois de ses côtés (65, 103a, 103b), à la manière d'une poche accessible depuis un côté (104) resté libre.

5. Sac d'emballage (43P) selon la revendication 3, **caractérisé en ce qu'**il comprend:
- une paroi intermédiaire (95) entre lesdites parois latérales (86, 87), ladite paroi intermédiaire (95) divisant ledit volume de confinement en deux compartiments;
- une deuxième ligne d'ouverture détachable (96) gravée sur ledit volet (LMB) en malignement avec le bord de ladite paroi intermédiaire (95), en formant deux parties séparables (97, 98), une sur chacun des deux compartiments.

6. Sac d'emballage (32, 43P) selon la revendication 1, **caractérisé en ce que** le volet (36, LMB) et ladite bande (33) pour une préhension manuelle sont formés du même matériau de celui des parois.

7. Sac d'emballage (32, 43P) selon l'une quelconque des précédentes revendications, **caractérisé en ce qu'**il comprend des moyens réversibles (40) pour fixer le volet (36) à la paroi sous-jacente (32), sur ledit côté libre (18) du volet (36).

8. Procédé pour la fabrication du sac d'emballage selon la revendication 1, comprenant les étapes consistant à:
a) former une ligne préparée d'ouverture détachable (56) sur une feuille (43) rectangulaire, formée de préférence à partir d'un matériau sous forme de film mince, ladite ligne (56) étant disposée parallèlement à un côté (49) de ladite feuille (43);
b) superposer un volet rectangulaire (57, 61) à ladite ligne d'ouverture détachable (56) sur toute la longueur de la ligne de la ligne d'ouverture détachable (56),
c) plier sur elle-même ladite feuille (43), y compris le volet superposé (LMB), environ à la moitié de la longueur d'une dimension de la feuille, le long d'une ligne de pliage (44) disposée parallèlement à la ligne d'ouverture détachable (56), ainsi obtenant deux pièces d'accouplement (47, 48);
d) assembler les bords (45, 46) des deux pièces d'accouplement (47, 48) de la feuille (43) et le volet (LMB), dont les bords sont perpendiculaires à la ligne de pliage (44), au moyen d'un flexion ou un collage;
e) souder lesdites deux parties d'accouplement (47, 48) de la feuille (43) y compris le volet (LMB) le long d'une ligne de soudage (65) disposée parallèlement à la ligne d'ouverture détachable (56), ladite ligne de soudage (65) pouvant être correspondant à la ligne de pliage (44) ou étant disposée entre la ligne de pliage (44) et la ligne d'ouverture détachable (56), de manière à obtenir une bande (66) d'un manche, dans lequel se trouve une ouverture (54, 55) presque centrale, pour fournir une préhension manuelle.

9. Procédé selon la revendication 8, **caractérisé en ce qu'**une partie d'accouplement (47) est supérieure à l'autre (48) selon une marge (64) d'une longueur précédemment fixée, dans laquelle on peut percer deux trous (50, 51) pour une fourche de levage ultérieure.

10. Procédé selon la revendication 8, **caractérisé en ce que** ladite ligne d'ouverture détachable (56) a la même longueur de ladite ligne de pliage (44).

11. Procédé selon la revendication 8, **caractérisé en ce que** ledit volet (LMB) est supérieure à ladite ligne de pliage (44) et est soudé ou collé à la partie d'accouplement opposée (47) afin de renforcer ladite bande (66).

12. Procédé selon la revendication 8, **caractérisé en ce que** lorsque le volet (57) se termine sur la ligne de pliage (44), la bande (66) de poignée est soudée ou collée à l'une ou à l'autre partie accouplement (47, 48), à proximité de la ligne de soudage (65).

13. Procédé selon la revendication 8, il **caractérisé en ce qu'**il comprend en outre l'application d'une bande adhésive (60) à travers le côté libre (58) du volet (LMB) en contact avec la partie d'accouplement (48) sous-jacente, la longueur de ladite bande (60) se prolongeant sur une partie ou sur l'ensemble du côté libre (58).

14. Procédé selon la revendication 8, **caractérisé en ce qu'**il comprend en outre l'application d'une bande adhésive double entre le volet (LMB) et la partie d'accouplement (48) sous-jacente, au voisinage de la face libre (58) du volet (LMB).
